# EUROPEAN PATENT SPECIFICATION

(11) **EP 2 021 483 B1**
(45) Date of publication and mention of the grant of the patent: **06.01.2016**
(21) Application number: 06748076.4
(22) Date of filing: 05.05.2006
(51) Int. Cl.: A61K 39/12, A61K 39/145, C07K 14/005, C12N 15/86, A61K 39/00

(54) **BIOMOLECULE SURFACE DISPLAY AND USES THEREOF**
BIOMOLEKÜLOBERFLÄCHENPRÄSENTATION UND VERWENDUNGEN DAVON
AFFICHAGE DE LA SURFACE D'UNE BIOMOLÉCULE ET SES UTILISATIONS

(43) Date of publication of application: 11.02.2009
(60) Divisional application: 11187828.6
(73) Proprietor: Temasek Life Sciences Laboratory Limited, Singapore 117604 (SG)
(72) Inventor: KWANG, Jimmy, Singapore 127140 (SG); LU, Li Qun, Singapore 138769 (SG)
(74) Representative: Cabinet Plasseraud
(86) International application number: PCT/SG2006/000117
(87) International publication number: WO 2007/129984

(56) References cited:
- ABE TAKAYUKI ET AL: "Baculovirus induces an innate immune response and confers protection from lethal influenza virus infection in mice." JOURNAL OF IMMUNOLOGY (BALTIMORE, MD. : 1950) 1 AUG 2003, vol. 171, no. 3, 1 August 2003 (2003-08-01), pages 1133-1139, XP002400984 ISSN: 0022-1767
- KODIHALLI S ET AL: "DNA vaccine encoding hemagglutinin provides protective immunity against H5N1 influenza virus infection in mice." JOURNAL OF VIROLOGY. MAR 1999, vol. 73, no. 3, March 1999 (1999-03), pages 2094-2098, XP002400985 ISSN: 0022-538X
- LU LIQUN ET AL: "Baculovirus-mediated promoter assay and transcriptional analysis of white spot syndrome virus orf427 gene" VIROLOGY JOURNAL, BIOMED CENTRAL, LONDON, GB, vol. 2, no. 1, 23 August 2005 (2005-08-23), page 71, XP021010918 ISSN: 1743-422X
- ERNST W ET AL: "BACULOVIRUS SURFACE DISPLAY: CONSTRUCTION AND SCREENING OF A EUKARYOTIC EPITOPE LIBRARY" NUCLEIC ACIDS RESEARCH, OXFORD UNIVERSITY PRESS, SURREY, GB, vol. 26, no. 7, 1998, pages 1718-1723, XP000919156 ISSN: 0305-1048
- OPERSCHALL E ET AL: "Enhanced protection against viral infection by co-administration of plasmid DNA coding for viral antigen and cytokines in mice." JOURNAL OF CLINICAL VIROLOGY : THE OFFICIAL PUBLICATION OF THE PAN AMERICAN SOCIETY FOR CLINICAL VIROLOGY. JUN 1999, vol. 13, no. 1-2, June 1999 (1999-06), pages 17-27, XP002400986 ISSN: 1386-6532
- KODIHALLI S ET AL: "Strategies for inducing protection against avian influenza A virus subtypes with DNA vaccines" VACCINE, BUTTERWORTH SCIENTIFIC. GUILDFORD, GB, vol. 18, no. 23, May 2000 (2000-05), pages 2592-2599, XP004195924 ISSN: 0264-410X
- AOKI H ET AL: "Induction of antibodies in mice by a recombinant baculovirus expressing pseudorabies virus glycoprotein B in mammalian cells" VETERINARY MICROBIOLOGY, AMSTERDAM, NL, vol. 68, no. 3-4, 31 August 1999 (1999-08-31), pages 197-207, XP002224593 ISSN: 0378-1135
- ZE C ET AL: "Comparison of the ability of viral protein-expressing plasmid DNAs to protect against influenza" VACCINE, BUTTERWORTH SCIENTIFIC. GUILDFORD, GB, vol. 16, no. 16, October 1998 (1998-10), pages 1544-1549, XP004129207 ISSN: 0264-410X
- KODIHALLI S ET AL: "Cross-Protection among Lethal H5N2 Influenza Viruses Induced by DNA Vaccine to the Hemagglutinin" JOURNAL OF VIROLOGY, THE AMERICAN SOCIETY FOR MICROBIOLOGY, US, vol. 71, no. 5, May 1997 (1997-05), pages 3391-3396, XP002992280 ISSN: 0022-538X
- SIMECKOVA-ROSENBERG J ET AL: "Protection of mice against lethal viral infection by synthetic peptides corresponding to B- and T-cell recognition sites of influenza A hemagglutinin" VACCINE, BUTTERWORTH SCIENTIFIC. GUILDFORD, GB, vol. 13, no. 10, July 1995 (1995-07), pages 927-932, XP004057511 ISSN: 0264-410X
- GAO WENTAO ET AL: "Protection of mice and poultry from lethal H5N1 avian influenza virus through adenovirus-based immunization." JOURNAL OF VIROLOGY. FEB 2006, vol. 80, no. 4, February 2006 (2006-02), pages 1959-1964, XP002400987 ISSN: 0022-538X
- HE FANG ET AL: "WSSV ie1 promoter is more efficient than CMV promoter to express H5 hemagglutinin from influenza virus in baculovirus as a chicken vaccine", BMC MICROBIOLOGY, BIOMED CENTRAL LTD, GB, vol. 8, no. 1, 31 December 2008 (2008-12-31), page 238, XP021048174, ISSN: 1471-2180, DOI: 10.1186/1471-2180-8-238

## Description

### Technical Field

The present invention relates generally to biomolecule surface display systems, with particular application to display of membrane glycoproteins as vaccines. In particular, the present invention relates to methods, vaccines, immunological compositions and kits for treating and/or preventing avian influenza, and to associated methods for modulating an immune response to avian influenza virus. More particularly, the present invention relates to methods, vaccines, immunological compositions and kits for treating and/or preventing strain H5N1 avian influenza virus, and to associated methods for modulating an immune response to strain H5N1 avian influenza virus.

### Background Art

Avian ,influenza virus is a significant worldwide infectious disease of birds that is caused by type A strains of the influenza virus. All birds are thought to be susceptible to the disease, which can result in symptoms ranging from mild illness to a highly contagious and rapidly fatal pathogenesis, resulting in severe epidemics.

Fifteen subtypes of influenza virus are known to infect birds, thus providing an extensive reservoir of influenza viruses within bird populations. In particular, because migratory birds are naturally resistant to infection, they provide a potent vector for infection to other bird populations such as domestic poultry which are particularly susceptible to epidemics of rapidly fatal influenza. All outbreaks of the highly pathogenic form have been caused by influenza A viruses of subtypes H5 and H7.

Quarantine of infected areas and destruction of infected or exposed flocks are standard procedures aimed at preventing the spread of avian influenza virus. However, such measures are not only hampered by the high contagiousness, ease of transmission and long term stability of the virus outside of its natural host, but they also carry a heavy economic toll in terms of both implementation of such measures and destruction of birds.

In 1997 in Hong Kong, the H5N1 strain of avian influenza virus jumped directly from birds to humans, causing severe respiratory disease in 18 humans, of whom 6 died. This infection coincided with an epidemic in Hong Kong's poultry population of avian influenza virus caused by the same strain. A further outbreak in 2003 caused 2 cases and 1 death. Further cases of human infection with avian influenza virus have been observed in several countries including the Netherlands, Vietnam and China. In addition, more recent outbreaks of birds infected with the H5N1 strain have been reported in eastern Europe and particularly Turkey.

H5N1 is of particular concern because it mutates rapidly and is adept at acquiring genes from viruses infecting other animal species. In addition, isolates from H5N1 have high pathogenicity and can cause severe disease in humans. Birds surviving infection excrete virus for at least 10 days, both orally and in faeces, thereby facilitating further spread at live poultry markets and by migratory birds.

Data on the clinical progression of human infection with H5N1 avian influenza virus is very limited, and antiviral drugs, only some of which can be used for both treatment and prevention, also have limitations. Lu L. et al (Baculovirus mediated promoter assay and transcriptional analysis of white spot syndrome virus orf427 gene - 2005 - Virology Journal, 2:71-77) reports the use of iel promoter to control the expression of the WSSV orf427 gene. Abe Takayuki et al (Baculovirus induces an innate immune response and confers protection from lethal influenza virus infection in mice - 2003 - The Jounal of Immunology, 171: 1133-1139) disclose an expression vector comprising a nucleic acid encoding the influenza virus HA peptide and a chicken β-actin promoter.

The present invention is predicated on the development of biomolecule surface display systems, with particular application of such systems to display of membrane glycoproteins as vaccines. In particular, the present invention relates to methods, vaccines, immunological compositions and kits for treating and/or preventing avian influenza, and to associated methods for modulating an immune response to avian influenza virus.

### Summary

It is described a method for presenting or displaying a polypeptide, wherein said method comprises:
(a) inserting a nucleic acid encoding said polypeptide into a baculovirus expression vector, wherein said baculovirus expression vector comprises an *ie1* promoter from white spot syndrome virus;
(b) transfecting at least one host cell with said expression vector; and
(c) expressing said polypeptide from said expression vector
wherein said polypeptide is presented or displayed on the surface membrane of a baculovirus.

It is described a system for presenting or displaying a polypeptide, wherein said system comprises:
(a) a nucleic acid encoding said polypeptide inserted into a baculovirus expression vector, wherein said baculovirus expression vector comprises an *ie1* promoter from white spot syndrome virus;
(b) means for transfecting at least one host cell with said expression vector; and
(c) means for expressing said polypeptide from said expression vector
wherein said polypeptide is presented or displayed on the surface membrane of a baculovirus.

According to a first aspect of the present invention, there is provided a vaccine for the treatment or prevention of a disease in a subject, wherein said disease is associated with an avian influenza virus, and wherein said vaccine comprises an expression vector comprising a nucleic acid encoding a hemagglutinin peptide, such that in use said hemagglutinin peptide is expressed by said expression vector in said subject, and wherein the expression vector further comprise an *ie1* promoter from white spot syndrome virus operably linked to the nucleic acid encoding said hemagglutinin peptide.

The subject may be human, avian or porcine.

The avian influenza virus may be an H5N1 subtype of avian influenza virus.

The expression vector may comprise a baculovirus expression vector. The baculovirus expression vector may be pseudotyped with a vesicular stomatitis virus glycoprotein.

The hemagglutinin peptide may comprise an amino acid sequence derived from an H5N1 subtype of avian influenza virus.

According to another aspect of the present invention, there is provided a vaccine for the treatment or prevention of a disease in a subject, wherein said disease is associated with an avian influenza virus, and wherein said vaccine comprises an expression vector comprising:
(a) a nucleic acid encoding a hemagglutinin peptide; and
(b) an *ie1* promoter from white spot syndrome virus operably linked to the nucleic acid encoding a hemagglutinin peptide
such that in use said hemagglutinin peptide is expressed by said expression vector in said subject.

According to another aspect of the present invention, there is provided an expression vector, wherein said expression vector comprises:
(a) a nucleic acid encoding an antigenic peptide; and
(b) an *ie1* promoter from white spot syndrome virus operably linked to the nucleic acid encoding an antigenic peptide
wherein said antigenic peptide comprises a hemagglutinin peptide comprising an amino acid sequence derived from an H5N1 subtype of avian influenza virus.

According to another aspect of the present invention, there is provided the expression vector of the invention for use in the treatment or prevention of a disease in a subject,
wherein said disease is associated with an avian influenza virus, such that in use said antigenic peptide is expressed by said expression vector in said subject.

According to another aspect of the present invention, there is provided a host cell comprising the expression vector of the invention.

According to another aspect of the present invention, there is provided a composition comprising an immunopotentiating agent selected from the group consisting of the vaccine of the invention, the expression vector of the invention or the host cell of the invention, together with a pharmaceutically acceptable carrier, diluent or excipient.

The composition may optionally comprise an adjuvant.

According to another of the present invention, there is provided a vaccine comprising the expression vector of the invention, the host cell of the invention or the composition of the invention for the treatment or prevention of a disease in a subject, wherein said disease is associated with an avian influenza virus.

According to another aspect of the present invention, there is provided a method for modulating an immune response, wherein said method comprises administering to a subject an effective amount of an immunopotentiating agent selected from the group consisting of the vaccine of the invention, the expression vector of the invention, the host cell of the invention or the composition of the invention.

According to another aspect of the present invention, there is provided a method for the treatment or prevention of a disease associated with an avian influenza virus, wherein said method comprises administering to a subject an effective amount of an immunopotentiating agent selected from the group consisting of the vaccine of the invention, the expression vector of the invention, the host cell of the invention or the composition of the invention.

According to another aspect of the present invention, there is provided use of the vaccine of the invention, the expression vector of the invention, the host cell of the invention or the composition of the invention for the modulation of an immune response.

According to another aspect of the present invention, there is provided use of the vaccine of the invention, the expression vector of the invention, the host cell of the invention or the composition of the invention for the manufacture of a medicament for the treatment of a disease associated with an avian influenza virus.

It is described a kit for use in treating or preventing a disease in a subject, wherein said disease is associated with an avian influenza virus, and wherein said kit comprises the vaccine of the invention, the expression vector of the invention, the host cell of the invention or the composition of the invention.

### Brief Description of the Figures

The present invention will now be described, by way of example only, with reference to the following figures.

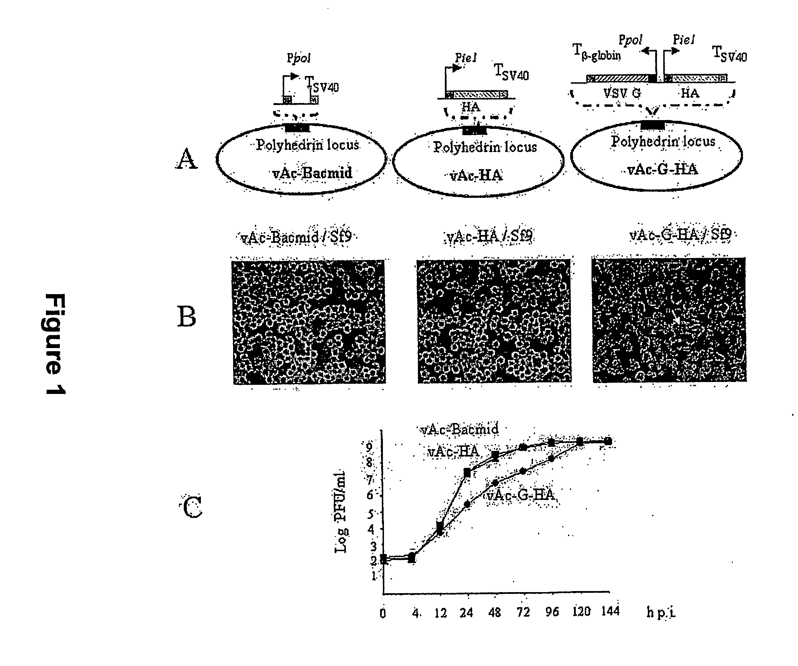

**Figure 1****.** Construction and production of recombinant baculoviruses. **(A)** Schematic representation of the genome of vAc-Bacmid, vAc-HA, and vAc-G-HA. The desired VSV G or HA expression cassettes were inserted within the polyhedrin locus through site-specific transposition employing a Bac-to-Bac system. **(B)** Syncytium formation in Sf9 cells infected with vAc-G-HA as indicated by white arrow. Images were captured at 72 h post transduction. **(C)** One-step growth curves. Each data point represents the mean value of three individual infections. Sf9 cells were infected by individual virus with a MOI of 0.5.

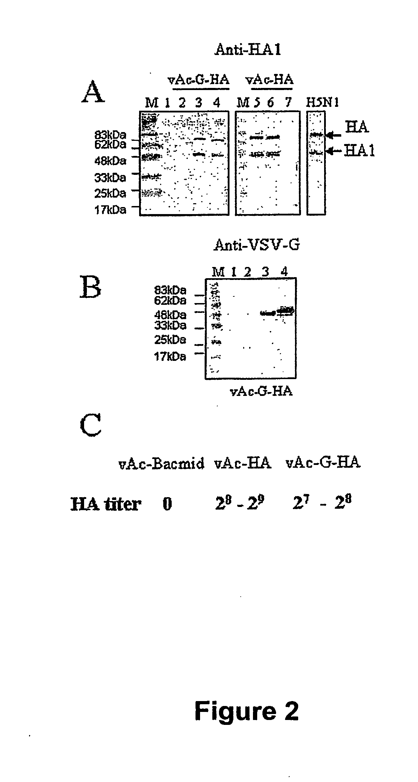

**Figure 2****.** Characterization of HA displayed on the surface of baculovirus vectors. **(A)** Membrane-association of HA in purified vAc-HA and vAc-G-HA particles. Purified H5N1 virions served as a positive control. Lane 1, purified vAc-Bacmid virions as negative control; lane 2, purified nucleocapsids from vAc-G-HA virions treated with Triton X-100; lane 3, complete vAc-G-HA virions; lane 4, total cellular extracts from Sf9 cells infected with vAc-G-HA; lane 5, total cellular extracts from Sf9 cells infected with vAc-G-HA; lane 6, complete vAc -HA virions; lane 7, purified nucleocapsids from vAc-HA virions treated with Triton X-100. **(B)** Membrane-association of VSV G in purified vAc-G-HA particles. Lanes 1-4 were the same samples as in (A). **(C)** Hemagglutination activity of HA-displaying baculoviruses. The hemagglutination titer was determined using serial twofold dilutions of purified vAc-G-HA particles and a 0.5% suspension of chicken erythrocytes per test well with a starting dilution of 1:2.

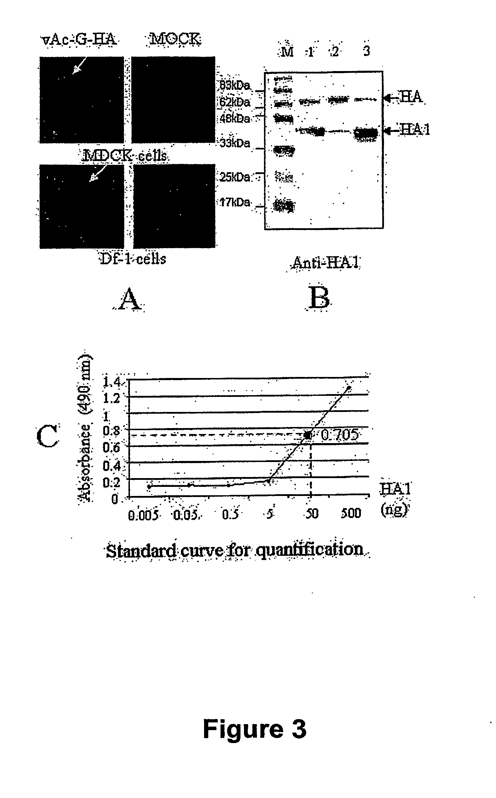

**Figure 3****. (A)** and **(B)** Transduction of the HA gene by vAc-G-HA. MDCK or Df-1 cells were transduced with vAc-G-HA at a MOI of 100. 16 h later, the cells were fixed for an IFA test or harvested for Western blot analysis. **(A)** Fluorescence micrographs of transduced mammalian cells in the IFA test with a HAl-specific monoclonal antibody. Arrows point to positive fluorescent cells. The fluorescence signal was detected using inverted fluorescence microscopy and the images were captured by a digital imaging system. **(B)** Detection of HA in both transduced cells through Western blot assay with anti-HAl serum. Lane 1, MDCK cellular extract; lane 2, Df-1 cellular extract; lane 3, purified H5N1 virions as positive control. (C) Quantification of the HA molecule displayed on vAc-G-HA virions through antigen capture ELISA. Purified IgG from guinea pigs and a monoclonal antibody were used as detector and capture antibodies, respectively, in the assay. 10-fold serially diluted HA1 protein was employed to construct a standard quantification curve as shown.

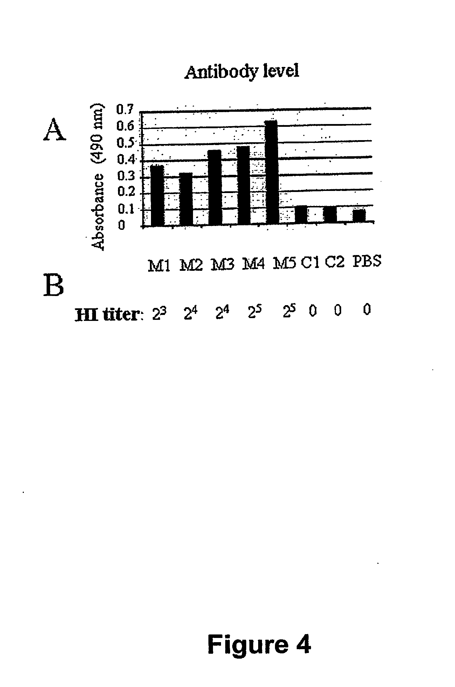

**Figure 4****. (A)** Antigenic analysis of vAc-G-HA. A group of 5 mice (M1- M5) were intramuscularly injected with vAc-G-HA particles. Two control mice (C1 and C2) were injected with equal amounts of purified vAc-Bacmid virions. The induced antibody was monitored by ELISA with HA1 protein coated as a capture antigen. Results are expressed as the mean absorbance of 1:10 diluted sera. **(B)** Serum HI titer for mice immunized with vAc-G-HA. The HI titer is expressed as the endpoint in a twofold dilution of sera.

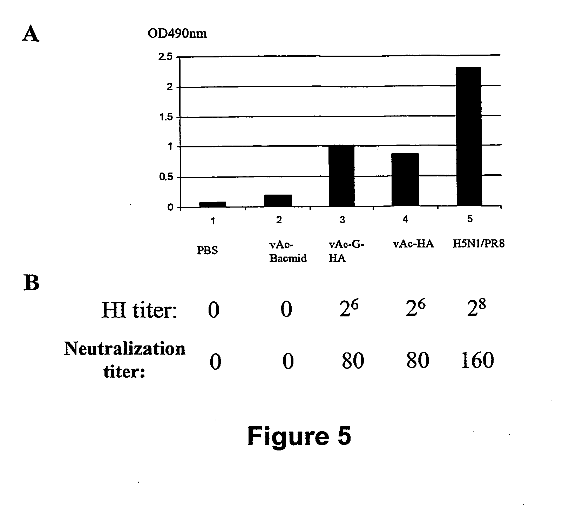

**Figure 5****. (A)** Antigenic analysis of HA-displaying baculoviruses. Three groups of 5 mice were intramuscularly injected with vAc-G-HA, vAc-HA, inactivated H5N1/PR8, and vAc-Bacmid individually. Pooled serum samples from each group were tested by ELISA with purified HA1 protein coated as the capture antigen. Results are expressed as the mean absorbance of 1:10 diluted sera. **(B)** HI assay and neutralization assay for the pooled serum samples from immunized mice. The HI titer is expressed as the endpoint in a two-fold dilution of sera. In the neutralization assay, the two-fold serial dilutions from 10 to 640 were mixed with an equal volume of virus diluent containing influenza virus at 2 x 10³ TCID₅₀/ml. The presence of viral protein was monitored by an IFA test with the polyclonal anti-HA1 antibody. The neutralization titer is expressed as the endpoint in the serial dilution.

### Definitions

As used herein, the term "comprising" means "including principally, but not necessarily solely". Furthermore, variations of the word "comprising", such as "comprise" and "comprises", have correspondingly varied meanings.

As used herein the terms "treating" and "treatment" refer to any and all uses which remedy a condition or symptoms, prevent the establishment of a condition or disease, or otherwise prevent, hinder, retard, ameliorate or reverse the progression of a condition or disease or other undesirable symptoms in any way whatsoever.

As used herein the term "effective amount" includes within its meaning a non-toxic but sufficient amount of an agent or compound to provide the desired effect. The exact amount required will vary from subject to subject depending on factors such as the species being treated, the age and general condition of the subject, the severity of the condition being treated, the particular agent being administered and the mode of administration and so forth. Thus, it is not possible to specify an exact "effective amount". However, for any given case, an appropriate "effective amount" may be determined by one of ordinary skill in the art using only routine experimentation.

As used herein, the terms "polypeptide", "peptide" and "protein" are used interchangeably to refer to a polymer of amino acid residues and to fragments, variants, analogues, orthologues or homologues thereof. Thus, these terms apply both to amino acid polymers in which one or more amino acid residues is a synthetic non-naturally occurring amino acid, such as a chemical analogue of a corresponding naturally occurring amino acid, as well as to naturally-occurring amino acid polymers.

As used herein, the terms "polynucleotide" or "nucleic acid" are used interchangeably and designate a molecule comprising one or more nucleotides, or an oligonucleotide, or a fragment thereof, including but not limited to RNA or DNA nucleotides or combinations thereof.

Within the scope of the terms "protein", "polypeptide", "peptide", "polynucleotide" and "nucleic acid" as used herein are fragments and variants thereof, including but not limited to reverse compliment and antisense forms of polynucleotides and nucleic acids.

The term "fragment" refers to a polynucleotide or polypeptide sequence that encodes a constituent or is a constituent of a full-length protein or gene. In terms of the polypeptide, the fragment may possess qualitative biological activity in common with the full-length protein.

The term "variant" as used herein refers to substantially similar sequences. Generally, nucleic acid sequence variants may encode polypeptides which possess qualitative biological activity in common. Generally, polypeptide sequence variants may also possess qualitative biological activity in common. Further, these polypeptide sequence variants may share at least 50%, 55%, 60%, 65%, 70%, 75%, 80%, 85%, 90%, 95%, 96%, 97%, 98% or 99% sequence identity.

Further, a variant polypeptide may include analogues, wherein the term "analogue" means a polypeptide which is a derivative of the disclosed polypeptides, which derivative comprises addition, deletion or substitution of one or more amino acids, such that the polypeptide retains substantially the same function as the native polypeptide from which it is derived.

As used herein, the term "expression vector" means a nucleic acid that has the ability confer expression of a nucleic acid fragment to which it is operably linked, in a cell or cell-free expression system. Within the context of the present invention, it is to be understood that an expression vector that comprises a promoter as defined herein may be a plasmid, bacteriophage, phagemid, cosmid, virus sub-genomic or genomic fragment, or other nucleic acid capable of maintaining and or replicating heterologous DNA in an expressible format should it be introduced into a cell.

As used herein, the term "operably linked" refers to transcriptional and translational regulatory polynucleotides that are positioned relative to a polypeptide-encoding polynucleotide in such a manner such that the polynucleotide is transcribed and the polypeptide is translated.

As used herein, the term "promoter" includes transcriptional regulatory sequences of a genomic gene, including the TATA box or initiator element, which may be required for accurate transcription initiation, with or without additional regulatory elements which alter gene expression in response to developmental and/or external stimuli, or in a tissue-specific manner. In the present context, the term "promoter" is also used to describe a recombinant, synthetic or fusion molecule, or derivative which confers, activates or enhances the expression of a nucleic acid molecule to which it is operably linked, and which encodes a peptide.

As used herein, the phrase "avian influenza virus" includes any virus causing or suspected of causing a disease in subjects including but not limited to avian, porcine or human subjects. In particular, "avian influenza virus" includes all types of avian influenza virus, including but not limited to type A, and all subtypes of avian influenza virus, including but not limited to subtypes H5 (in particular H5N1 and H5N2) and H7 (in particular H7N1).

As used herein, the phrase "disease associated with an avian influenza virus" means any disease, disease state or disorder caused by or associated with an avian influenza virus.

As used herein, the term "modulating" when used in relation to an immune response means increasing or decreasing, either directly or indirectly, an immune response against an antigen.

### Best Mode of Performing the Invention

The inventors have developed a biomolecule surface display system, with particular application to display of membrane glycoproteins as vaccines. In particular, the inventors have demonstrated the application of a baculovirus expression system as an immunizing reagent against influenza virus through synergistic surface display and gene transduction of the viral hemagglutinin (HA) protein. The efficient display of immunodominant viral membrane HA protein on the surface of recombinant baculovirus, along with the VSV G protein, confers a major advantage in vaccination strategies against influenza virus. In addition to application of this baculovirus expression system as a vaccination vector, the viral membrane display system can also be used to characterize the structure and function of a wide variety of viral membrane glycoproteins.

Under the control of the *ie1* promoter from white spot syndrome virus, the HA gene of H5N1 influenza virus was efficiently expressed in both insect and mammalian cells using baculovirus vectors. Concurrent display of HA with vesicular stomatitis virus glycoprotein did not reduce the efficiency of HA display on the baculoviral surface. The partially cleaved HA protein was displayed on the surface of baculovirus, thereby conferring viral particle hemagglutination activity. Intramuscular injection of the purified HA-displaying baculovirus into mice stimulated the production of antibodies with hemagglutination inhibition activities. This baculovirus expression system therefore harbors the antigenic HA peptide as a structural protein and sustains the ability to express the peptide through *in vivo* transduction.

### Methods, kits and systems for presenting or displaying biomolecules

The present invention discloses biomolecule surface display systems, with particular application of such systems to display of membrane glycoproteins as vaccines. As will be apparent to persons of skill in the art, these biomolecule surface display systems are not limited to the particular applications disclosed herein, but find broad application in any situation in which it is desirable to present a biomolecule, and in particular, a membrane glycoprotein.

Accordingly, in one embodiment, the present description discloses methods, kits and systems for vaccine production involving surface display of membrane glycoproteins. These methods, kits and systems may utilize the baculovirus-based surface display systems exemplified herein in relation to production of a vaccine against strain H5N1 avian influenza virus. However, persons of skill in the art will recognize and understand that the methods, kits and systems for vaccine production involving surface display of membrane glycoproteins are not limited to production of the vaccines disclosed herein.

Accordingly, it is described methods for presenting or displaying a polypeptide, wherein said methods comprise:
(a) inserting a nucleic acid encoding said polypeptide into a baculovirus expression vector, wherein said baculovirus expression vector comprises an *ie1* promoter from white spot syndrome virus;
(b) transfecting at least one host cell with said expression vector; and
(c) expressing said polypeptide from said expression vector
wherein said polypeptide is presented or displayed on the surface membrane of a baculovirus.

The present invention further provides systems for presenting or displaying a polypeptide, wherein said systems comprise:
(a) a nucleic acid encoding said polypeptide inserted into a baculovirus expression vector, wherein said baculovirus expression vector comprises an *ie1* promoter from white spot syndrome virus;
(b) means for transfecting at least one host cell with said expression vector; and
(c) means for expressing said polypeptide from said expression vector
wherein said polypeptide is presented or displayed on the surface membrane of a baculovirus.

### Vaccines

The present invention also provides vaccines for the treatment or prevention of a disease in a subject, wherein said disease is associated with an avian influenza virus, and wherein said vaccine comprises an expression vector comprising a nucleic acid encoding a hemagglutinin peptide, such that in use said hemagglutinin peptide is expressed by said expression vector in said subject, wherein the expression vector further comprises an *ie1* promoter from white spot syndrome virus operably linked to the nucleic acid encoding said hemagglutinin peptide.

The subject may be human, avian or porcine.

The avian influenza virus may be an H5N1 subtype of avian influenza virus.

The expression vector may comprise a baculovirus expression vector. The baculovirus expression vector may be pseudotyped with a vesicular stomatitis virus glycoprotein.

The promoter is operably linked to the nucleic acid encoding an hemagglutinin peptide.

The hemagglutinin peptide may comprise an amino acid sequence derived from an H5N1 subtype of avian influenza virus.

The present invention also provides vaccines comprising the expression vectors, host cells or compositions as herein described, for the treatment or prevention of a disease in a subject, wherein said disease is associated with an avian influenza virus.

### Expression vectors and host cells

The present invention also provides expression vectors comprising a nucleic acid encoding an antigenic peptide, wherein said antigenic peptide comprises a hemagglutinin peptide comprising an amino acid sequence derived from an H5N1 subtype of avian influenza virus.

The present invention further provides expression vectors comprising a nucleic acid encoding an antigenic peptide, for use in the treatment or prevention of a disease in a subject, wherein said disease is associated with an avian influenza virus, such that in use said antigenic peptide is expressed by said expression vector in said subject.

The present invention also provides host cells comprising the expression vectors as described herein.

### Compositions and immunopotentiating agents

The present invention contemplates compositions comprising an immunopotentiating agent selected from the group consisting of the vaccines, expression vectors or host cells as described herein, together with a pharmaceutically acceptable carrier, diluent or excipient

The immunopotentiating agents may be formulated into a composition as neutral or salt forms. Pharmaceutically acceptable salts include the acid addition salts (formed with free amino groups of the peptide) and which are formed with inorganic acids such as, for example, hydrochloric or phosphoric acids, or such organic acids such as acetic, oxalic, tartaric, maleic, and the like. Salts formed with the free carboxyl groups may also be derived from inorganic basis such as, for example, sodium, potassium, ammonium, calcium, or ferric hydroxides, and such organic basis as isopropylamine, trimethylamine, 2-ethylamino ethanol, histidine, procaine, and the like.

In general, suitable compositions may be prepared according to methods which are known to those of ordinary skill in the art and may include pharmaceutically acceptable diluents, adjuvants and/or excipients. The diluents, adjuvants and excipients must be "acceptable" in terms of being compatible with the other ingredients of the composition, and not deleterious to the recipient thereof.

Examples of pharmaceutically acceptable diluents are demineralised or distilled water; saline solution; vegetable based oils such as peanut oil, safflower oil, olive oil, cottonseed oil, maize oil, sesame oils such as peanut oil, safflower oil, olive oil, cottonseed oil, maize oil, sesame oil, arachis oil or coconut oil; silicone oils, including polysiloxanes, such as methyl polysiloxane, phenyl polysiloxane and methylphenyl polysolpoxane; volatile silicones; mineral oils such as liquid paraffin, soft paraffin or squalane; cellulose derivatives such as methyl cellulose, ethyl cellulose, carboxymethylcellulose, sodium carboxymethylcellulose or hydroxypropylmethylcellulose; lower alkanols, for example ethanol or iso-propanol; lower aralkanols; lower polyalkylene glycols or lower alkylene glycols, for example polyethylene glycol, polypropylene glycol, ethylene glycol, propylene glycol, 1,3-butylene glycol or glycerin; fatty acid esters such as isopropyl palmitate, isopropyl myristate or ethyl oleate; polyvinylpyrridone; agar; carrageenan; gum tragacanth or gum acacia, and petroleum jelly. Typically, the carrier or carriers will form from 1% to 99.9% by weight of the compositions. Most preferably, the diluent is saline.

For administration as an injectable solution or suspension, non-toxic parenterally acceptable diluents or carriers can include, Ringer's solution, medium chain triglyceride (MCT), isotonic saline, phosphate buffered saline, ethanol and 1,2 propylene glycol.

Some examples of suitable carriers, diluents, excipients and adjuvants for oral use include peanut oil, liquid paraffin, sodium carboxymethylcellulose, methylcellulose, sodium alginate, gum acacia, gum tragacanth, dextrose, sucrose, sorbitol, mannitol, gelatine and lecithin. In addition these oral formulations may contain suitable flavouring and colourings agents. When used in capsule form the capsules may be coated with compounds such as glyceryl monostearate or glyceryl distearate which delay disintegration.

Adjuvants typically include emollients, emulsifiers, thickening agents, preservatives, bactericides and buffering agents.

Solid forms for oral administration may contain binders acceptable in human and veterinary pharmaceutical practice, sweeteners, disintegrating agents, diluents, flavourings, coating agents, preservatives, lubricants and/or time delay agents. Suitable binders include gum acacia, gelatine, corn starch, gum tragacanth, sodium alginate, carboxymethylcellulose or polyethylene glycol. Suitable sweeteners include sucrose, lactose, glucose, aspartame or saccharine. Suitable disintegrating agents include corn starch, methylcellulose, polyvinylpyrrolidone, guar gum, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc.

Liquid forms for oral administration may contain, in addition to the above agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides or mixtures thereof.

Suspensions for oral administration may further comprise dispersing agents and/or suspending agents. Suitable suspending agents include sodium carboxymethylcellulose, methylcellulose, hydroxypropylmethyl-cellulose, poly-vinyl-pyrrolidone, sodium alginate or acetyl alcohol. Suitable dispersing agents include lecithin, polyoxyethylene esters of fatty acids such as stearic acid, polyoxyethylene sorbitol mono- or di-oleate, -stearate or - laurate, polyoxyethylene sorbitan mono- or di-oleate, -stearate or -laurate and the like.

Emulsions for oral administration may further comprise one or more emulsifying agents. Suitable emulsifying agents include dispersing agents as exemplified above or natural gums such as guar gum, gum acacia or gum tragacanth.

Methods for preparing parenterally administrable compositions are apparent to those skilled in the art, and are described in more detail in, for example, Remington's Pharmaceutical Science, 15th ed., Mack Publishing Company, Easton, Pa.

The composition may incorporate any suitable surfactant such as an anionic, cationic or non-ionic surfactant such as sorbitan esters or polyoxyethylene derivatives thereof. Suspending agents such as natural gums, cellulose derivatives or inorganic materials such as silicaceous silicas, and other ingredients such as lanolin, may also be included.

One or more immunopotentiating agents can be used as actives in the preparation of immunopotentiating compositions. Such preparation uses routine methods known to persons skilled in the art. Typically, such compositions are prepared as injectables, either as liquid solutions or suspensions; solid forms suitable for solution in, or suspension in, liquid prior to injection may also be prepared. The preparation may also be emulsified. The active immunogenic ingredients are often mixed with excipients that are pharmaceutically acceptable and compatible with the active ingredient.

### Routes of administration

According to the methods of present invention, vaccines and compositions may be administered by any suitable route, either systemically, regionally or locally. The particular route of administration to be used in any given circumstance will depend on a number of factors, including the nature of the disease to be treated, the severity and extent of the disease, the required dosage of the particular compounds to be delivered and the potential side-effects of the desired vaccines or compositions.

For example, in circumstances where it is required that appropriate concentrations of the desired vaccines or compositions are delivered directly to the site to be treated, administration may be regional rather than systemic. Regional administration provides the capability of delivering very high local concentrations of the desired vaccines or compositions to the required site and thus is suitable for achieving the desired therapeutic or preventative effect whilst avoiding exposure of other organs of the body to the vaccines or compositions and thereby potentially reducing side effects.

By way of example, administration according to embodiments of the invention may be achieved by any standard routes, including intracavitary, intravesical, intramuscular, intraarterial, intravenous, subcutaneous, topical or oral. Intracavitary administration may be intraperitoneal or intrapleural.

If desired, devices or compositions containing the immunopotentiating agents suitable for sustained or intermittent release could be, in effect, implanted in the body or topically applied thereto for the relatively slow release of such materials into the body.

Administration of an expression vector or host cell may include delivery via direct oral intake, systemic injection, or delivery to selected tissue(s) or cells, or indirectly via delivery to cells isolated from a subject or a compatible donor.

With regard to nucleic acid based compositions, all modes of delivery of such compositions are contemplated by the present invention. Delivery of these compositions to cells or tissues of an animal may be facilitated by microprojectile bombardment, liposome mediated transfection (e.g., lipofectin or lipofectamine), electroporation, calcium phosphate or DEAE-dextran-mediated transfection, for example. In an alternate embodiment, a synthetic construct may be used as a therapeutic or prophylactic composition in the form of a "naked DNA" composition as is known in the art. A discussion of suitable delivery methods may be found in Chapter 9 of CURRENT PROTOCOLS IN MOLECULAR BIOLOGY (Eds. Ausubel et al.; John Wiley & Sons Inc., 1997 Edition) or on the Internet site DNAvaccine.com. The compositions may be administered by intradermal (e.g., using panjet™ delivery) or intramuscular routes.

The step of introducing the synthetic polynucleotide into a target cell will differ depending on the intended use and species, and can involve one or more of non-viral and viral vectors, cationic liposomes, retroviruses, and baculoviruses such as, for example, described in Mulligan, R.C., (1993 Science 260 926-932). Such methods can include, for example:
A. Local application of the synthetic polynucleotide by injection (Wolff et al., 1990, Science 247 1465-1468), surgical implantation, instillation or any other means. This method can also be used in combination with local application by injection, surgical implantation, instillation or any other means, of cells responsive to the protein encoded by the synthetic polynucleotide so as to increase the effectiveness of that treatment. This method can also be used in combination with local application by injection, surgical implantation, instillation or any other means, of another factor or factors required for the activity of said protein.
B. General systemic delivery by injection of DNA, (Calabretta et al., 1993, Cancer Treat. Rev. 19 169-179), or RNA, alone or in combination with liposomes (Zhu et al., 1993, Science 261 209-212), viral capsids or nanoparticles (Bertling et al., 1991, Biotech. Appl. Biochem. 13 390-405) or any other mediator of delivery. Improved targeting might be achieved by linking the synthetic polynucleotide to a targeting molecule (the so-called "magic bullet" approach employing, for example, an antibody), or by local application by injection, surgical implantation or any other means, of another factor or factors required for the activity of the protein encoding said synthetic polynucleotide , or of cells responsive to said protein.
C. Injection or implantation or delivery by any means, of cells that have been modified ex vivo by transfection (for example, in the presence of calcium phosphate: Chen et al., 1987, Mole. Cell Biochem. 7 2745-2752, or of cationic lipids and polyamines: Rose et al., 1991, BioTech. 10 520-525), infection, injection, electroporation (Shigekawa et al., 1988, BioTech. 6 742-751) or any other way so as to increase the expression of said synthetic polynucleotide in those cells. The modification can be mediated by plasmid, bacteriophage, cosmid, viral (such as adenoviral or retroviral; Mulligan, 1993, Science 260 926-932; Miller, 1992, Nature 357 455-460; Salmons et al., 1993, Hum. Gen. Ther. 4 129-141) or other vectors, or other agents of modification such as liposomes (Zhu et al., 1993, Science 261 209-212), viral capsids or nanoparticles (Bertling et al., 1991, Biotech. Appl. Biochem. 13 390-405), or any other mediator of modification. The use of cells as a delivery vehicle for genes or gene products has been described by Barr et al., 1991, Science 254 1507-1512 and by Dhawan et al., 1991, Science 254 1509-1512. Treated cells can be delivered in combination with any nutrient, growth factor, matrix or other agent that will promote their survival in the treated subject.

The compositions may also be administered in the form of liposomes. Liposomes are generally derived from phospholipids or other lipid substances, and are formed by mono-or multi-lamellar hydrated liquid crystals that are dispersed in an aqueous medium. Any non-toxic, physiologically acceptable and metabolisable lipid capable of forming liposomes can be used. The compositions in liposome form may contain stabilisers, preservatives, excipients and the like. The preferred lipids are the phospholipids and the phosphatidyl cholines (lecithins), both natural and synthetic. Methods to form liposomes are known in the art, and in relation to this specific reference is made to: Prescott, Ed., Methods in Cell Biology, Volume XIV, Academic Press, New York, N.Y. (1976), p. 33 *et seq.*

### Dosages

The effective dose level of the administered compound for any particular subject will depend upon a variety of factors including: the type of disease being treated and the stage of the disease; the activity of the compound employed; the composition employed; the age, body weight, general health, sex and diet of the subject; the time of administration; the route of administration; the rate of sequestration of compounds; the duration of the treatment; drugs used in combination or coincidental with the treatment, together with other related factors well known in the art.

One skilled in the art would be able, by routine experimentation, to determine an effective, non-toxic dosage which would be required to treat applicable conditions. These will most often be determined on a case-by-case basis.

In terms of weight, a therapeutically effective dosage of a composition for administration to a patient is expected to be in the range of about 0.01mg to about 150mg per kg body weight per 24 hours; typically, about 0.1mg to about 150mg per kg body weight per 24 hours; about 0.1mg to about 100mg per kg body weight per 24 hours; about 0.5mg to about 100mg per kg body weight per 24 hours; or about 1.0mg to about 100mg per kg body weight per 24 hours. More typically, an effective dose range is expected to be in the range of about 5mg to about 50mg per kg body weight per 24 hours.

Alternatively, an effective dosage may be up to about 5000mg/m². Generally, an effective dosage is expected to be in the range of about 10 to about 5000mg/m², typically about 10 to about 2500mg/m², about 25 to about 2000mg/m², about 50 to about 1500mg/m², about 50 to about 1000mg/m², or about 75 to about 600mg/m².

Further, it will be apparent to one of ordinary skill in the art that the optimal quantity and spacing of individual dosages will be determined by the nature and extent of the condition being treated, the form, route and site of administration, and the nature of the particular individual being treated. Also, such optimum conditions can be determined by conventional techniques.

It will also be apparent to one of ordinary skill in the art that the optimal course of treatment, such as, the number of doses of the composition given per unit time, can be ascertained by those skilled in the art using conventional course of treatment determination tests.

### Methods of treatment

Also contemplated by the present invention are methods for modulating an immune response, wherein said methods comprise administering to a subject an effective amount of an immunopotentiating agent selected from the group consisting of the vaccines, expression vectors and host cells as described herein.

In addition, the present invention provides methods for the treatment or prevention of a disease associated with an avian influenza virus, wherein said methods comprise administering to a subject an effective amount of an immunopotentiating agent selected from the group consisting of the vaccines, expression vectors and host cells as described herein.

### Assessment of immunisation efficacy

The effectiveness of an immunisation undertaken in accordance with the methods of the present invention may be assessed using any suitable technique. For example, CTL lysis assays may be employed using stimulated splenocytes or peripheral blood mononuclear cells (PBMC) on peptide coated or recombinant virus infected cells using ⁵¹Cr labelled target cells. Such assays can be performed using, for example, primate, mouse or human cells (Allen et al., 2000, J. Immunol. 164(9): 4968-4978 also Woodberry *et al.,* infra). Alternatively, the efficacy of the immunisation may be monitored using one or more techniques including, but not limited to, HLA class I Tetramer staining of both fresh and stimulated PBMCs (see for example Allen et al., supra), proliferation assays (Allen *et al.,* supra), Elispot™ Assays and intracellular INF-gamma staining (Allen *et al.,* supra), ELISA Assays for linear B cell responses, and Western blots of cell samples expressing the antigenic peptides as described herein.

### Kits

It is described kits for use in treating or preventing a disease in a subject, wherein said disease is associated with an avian influenza virus, and wherein said kit comprises the vaccines, expression vectors, host cells or compositions as described herein.

Typically, kits for carrying out a method of the invention contain all the necessary reagents to carry out the method. Typically, the kits will comprise one or more containers, containing for example, wash reagents, and/or other reagents capable of releasing a bound component from a polypeptide or fragment thereof.

In the context of the present description, a compartmentalised kit includes any kit in which reagents are contained in separate containers, and may include small glass containers, plastic containers or strips of plastic or paper. Such containers may allow the efficient transfer of reagents from one compartment to another compartment whilst avoiding cross-contamination of the samples and reagents, and the addition of agents or solutions of each container from one compartment to another in a quantitative fashion. Such kits may also include a container which will accept a test sample, a container which contains the polymers used in the assay and containers which contain wash reagents (such as phosphate buffered saline, Tris-buffers, and like).

Typically, a kit of the present invention will also include instructions for using the kit components to conduct the appropriate methods.

The present invention will now be described by reference to the following examples, which should not be construed in any way as limiting the scope of the invention.

### Examples

### Example 1: Construction of recombinant baculovirus HA-expression casettes

Hemagglutinin (HA) of H5N1 influenza virus is one of two major membrane viral glycoproteins responsible for the production of neutralization antibodies (Bosch, et al., 1979, Kawaoka and Webster 1988). To determine the potential of generating a baculovirus-based vaccine against H5N1 virus (A/Goose/Guangdong/3/97/H5N1), the inventors constructed two recombinant baculovirus expression vectors, each containing a HA-expression cassette. The first baculovirus expression vector was produced with the HA-expression cassette under the control of the white spot syndrome virus (WSSV) *ie1* promoter, named vAc-HA (Fig. 1A). The second baculovirus expression vector was produced with the HA-expression cassette and an additional vesicular stomatitis virus glycoprotein (VSV G)-expression cassette under the control of the baculovirus polyhedrin promoter, named vAc-G-HA (Fig. 1A).

For the generation of the recombinant baculovirus vectors, the AcMNPV polyhedrin promoter-controlled VSV G expression cassette or WSSV *ie1* promoter-controlled HA expression cassette were inserted into the shuttle vector pFastBac1 and integrated into the baculovirus genome within DH10BAC™ according to the protocol of the Bac-To-Bac system (Invitrogen). A control virus, vAc-Bacmid, was also constructed by integrating an empty pFastBac1 vector into the bacmid genome (Fig. 1A).

The desired VSV G or HA expression cassettes were inserted within the polyhedrin locus through site-specific transposition employing the Bac-to-Bac system. VSV G cDNA with a β-globin terminator was then PCR amplified from pVSV-G with the primers 5'-CGCGGATCCATGAAGTGCCTTTTGTACTTAG-3' (SEQ ID NO: 1) and 5'-CCCAAGCTTCCAACACACTATTGCAATGAA-3' (SEQ ID NO: 2). VSV G cDNA was placed downstream of the polyhedrin promoter, which was PCR amplified from a bacmid genome with the primers 5'-TCCCCCGGGGGATGGTTGGCTACGTATACTCCG-3' (SEQ ID NO: 3) and 5'-CGCGGATCCGGTTTCGGACCGAGATCCGC-3' (SEQ ID NO: 4). The HA cDNA was amplified from the genome of the H5N1 virus through RT-PCR with a primer set of 5'-ATAAGAATGCGGCCGCTATGGAGAAAACAGTGCTTCTTCTTG-3' (SEQ ID NO: 5) and 5'-CCGCTCGAGCGGTTAAATGCAAATTCTGCATTGTAACGATC-3' (SEQ ID NO: 6). HA cDNA was then placed upstream of the SV4O terminator in a pFastBac1 vector and downstream of the *ie1* promoter, which was amplified from the WSSV genome (Lu *et al.,* 2005) with a primer set of 5'-TCCCTACGTATCAATTTTATGTGGCTAATGGAGA-3' (SEQ ID NO: 7) and 5'-ACGCGTCGACCTTGAGTGGAGAGAGAGCTAGTTATAA-3' (SEQ ID NO: 8).

Infection of insect Sf9 cells with vAc-G-HA resulted in extensive cell-cell fusion (Fig. 1B). The phenotype was due to the very high expression level of VSV G protein with membrane-fusion activity by the polyhedrin promoter.

The kinetics of virion production in Sf9 cells was also examined by generating a one-step growth curve of infectious virus production (Fig. 1C). The temporal kinetics of the growth curves (Lu *et al.,* 2003) of these viruses were similar, although peak virion production of vAc-G-HA lagged behind those of vAc-HA and control vAc-Bacmid. Titer of vAc-G-HA increased to 10⁹ PFU (plaque forming unit)/ml at 120 h p.i., while vAc-HA and vAc-Bacmid generated the same titer at 96 h p.i. (Fig. 1C). The extensive syncytium formation of Sf9 cells infected with vAc-G-HA might delay the release of virions in the late infection.

### Example 2: Membrane display of hemagglutinin

The inventors purified vAc-HA, vAc-G-HA and vAc-Bacmid from the supernatant of infected insect cells by ultracentrifugation. To investigate whether HA was displayed on the membrane of vAc-HA and vAc-G-HA, the purified virions were treated with 1% Triton X-100 for 15 minutes to disrupt the viral membrane structure, and then viral nucleocapsids were collected through ultracentrifugation. To produce antibody against HA protein, His₆-tagged HA1 was expressed and purified from baculovirus-infected SF9 cells, with polyclonal antibody against HA protein raised in guines pigs using His₆-tagged HA1 as antigen. Western blot analysis showed that HA was detected in the purified virions before treatment with Triton X-100, and not detected in the collected pellet comprised of only viral nucleocapsids (Fig. 2A).

It was found that the HA was partially cleaved to HA1 and HA2 in both the infected SF9 cells and the purified vAc-G-HA particles, being similar to that of the purified H5N1 virions (Fig. 2A). These data suggested that the baculovirus vector could display HA-like viral glycoproteins, with similar post-translational modification to its natural products. Western blot assays using an anti-VSV G monoclonal antibody also indicated that VSV G was a membrane-associated structural component in purified vAc-G-HA virions (Fig. 2B).

To determine whether the baculovirus surface-displayed HA protein sustained authentic biological property, the hemagglutination activity of purified virions of vAc-HA and vAc-G-HA was tested (Fig. 2C). Each 25 µl of vAc-HA or vAc-G-HA at a titer of 10¹⁰ PFU/ml gave a hemagglutination titer of around 2⁸ using a standard hemagglutination assay (Wood *et al.,* 1985). The results indicated that co-display of VSV G with HA would not significantly reduce the displaying efficiency of HA on the baculovirus membrane.

### Example 3: Transduction ability of vAc-G-HA

As VSV G confers a transduction advantage of vAc-G-HA over vAc-HA, *in vivo* vaccination experiments were undertaken to assess the transduction ability of vAc-G-HA. A transduction assay was performed (Hsu *et al.,* 2004) in the cell lines (1) canine MDCK and (2) chicken Df-1 cells. Fig. 3A shows that vAc-G-HA efficiently transduced these cell lines as indicated by the positive fluorescent cells. The expression was further confirmed by western blot analysis of the total cellular extract with anti-HA1 serum raised in guinea pigs, as shown in Fig. 3B.

To further monitor the quantity of the HA molecule displayed on the surface of vAc-G-HA, an antigen-capture ELISA was developed based on the guinea pig anti-HA1 polyclonal antiserum and a HA1 monoclonal antibody, as reported previously (He *et al.,* 2005).

His₆-tagged HA1 was expressed and purified from baculovirus infected SF9 cells. To construct the HA1-expressing baculovirus with a pFast HTa vector of the Bac-To-Bac system, HA1 coding sequence was amplified from the HA cDNA described above with a primer set of 5'-CGCGGATCCGATGGAGAAAACAGTGCTTCTTCTTTG-3' (SEQ ID NO: 9) and 5'-CGGGGTACCCTCTCTTTGAGGGGTATTTCT-3' (SEQ ID NO: 10). For protein expression, Sf9 cells were infected with the recombinant virus at a MOI of 5 and harvested at 72 h p.i. Purification of His₆-tagged HA1 protein from the total cellular extract was performed with a Ni²⁺ charged resin slurry.

The antigen capture ELISA was designed by coating microtiter plates with purified IgG from guinea pigs. The bound antigen was then detected with hybridoma supernatants containing HA1-specific monoclonal antibodies. 10-fold serially diluted HA1 protein was employed to construct a standard quantification curve. The amount of HA1 in the vAc-G-HA sample was determined as the point value in the standard curve that represents the same absorbance at 490 nm (Fig. 3C). From this curve, each 25 µl of vAc-G-HA at a titer of 10¹⁰ PFU/ml was found to contain about 50 ng HA1. These findings indicated that the HA protein sustained authentic hemagglutination activity and was efficiently displayed on the surface of the baculovirus.

### Example 4: Immunogenicity of vAc-G-HA

The immunogenicity of vAc-G-HA was then investigated through intra-muscular immunization of 6-8 week BALB/c mice with purified vAc-G-HA virions. At 4 weeks after vaccination with 5 x 10⁹ PFU of vAc-G-HA particles, the mice were boosted with the same amount of virions. One week later, the mice were bled through the retro-orbital plexus and serum from each animal was tested individually for antibodies against HA by ELISA and hemagglutination inhibition (HI) assays (Wood *et al.,* 1985).

The ELISA test was set up to monitor the HA-specific antibody level by coating 96-well microplates with 60 ng of purified His₆-tagged HA1 protein per well. As shown in Fig. 4A, all 5 immunized mice developed significant antibody responses, as detected by the ELISA in which purified HA1 protein was coated as antigen. The two control mice immunized with the vAc-Bacmid displayed only background sera titers.

The vAc-G-HA induced serum HI antibody responses in all 5 immunized mice, with a titer in the range of 2³ to 2⁵ (Fig. 4B). The HI antibody responses were comparable to those mice vaccinated intranasally with live H5 influenza viruses that demonstrated a titer in the range of 2⁴ to 2⁷, as reported by Takada *et al.* (1999). The specific and significant antibody response from mice inoculated with vAc-G-HA indicated that the HA molecule displayed on the viral surface or expressed through viral transduction sustained its antigenicity.

In order to further characterize the immunogenicity of vAc-G-HA and vAc-HA, 7 week BALB/c mice (five mice each group) were intra-muscularly immunized with purified virions. The inactivated H5N1/PR8 vaccine strain amplified from MDCK cells was used as a positive control, with vAc-Bacmid vaccinated mice being used as a negative control. At 4 weeks after intramuscular vaccination with 256 HA units of purified baculovirus particles or inactivated H5N1/PR8 virus, mice were boosted with the same amount of virions. One week later, the mice were bled through the retro-orbital plexus, and the pooled serum from each group of 5 mice was tested individually for antibodies against HA by ELISA as described by He *et al.,* 2005 and by Hemagglutination Inhibition (HI) assays as described by Wood *et al.,* 1985. ELISAs were set up to monitor the HA-specific antibody levels by coating 96-well microplates with 60 ng purified His6-tagged HA1 protein per well.

As shown in Fig. 5A, mice immunized with vAc-HA or vAc-G-HA developed significant antibody responses as detected by ELISA in which purified HA1 protein was coated as the antigen. Mice immunized with vAc-Bacmid displayed only background sera titers. Both vAc-G-HA and vAc-HA induced serum HI antibody responses in the immunized mice, with a titer of 2⁶ (Fig. 5B). The HI titers of baculovirus vectors were comparable to those mice vaccinated with the vaccine strain H5N1/PR8 that has an average titer of 2⁸ (Fig. 5B).

In the ELISA test, antibody responses induced by vAc-G-HA were around 18% higher than that of vAc-HA. Surprisingly, the H5N1/PR8 vaccine strain induced higher levels of antibody response as shown by the ELISA. Accordingly, the induced anti-sera demonstrated four fold higher HI activity than the HA-displaying baculoviruses (Fig. 5A and 5B). The specific and significant antibody response from the baculovirus-vaccinated mice indicated that the HA molecule displayed on the viral surface or expressed through viral transduction sustained its authentic antigenicity.

The *in vitro* neutralization ability of the induced antibody was then tested by the HA-displaying baculoviruses. A standard microneutralization test based on the H5N1/PR8 and MDCK cell system was performed as reported previously by Rowe *et al.,* 1999. The sera induced by both HA-displaying baculoviruses showed a significant neutrlization titer of 80, while the H5N1/PR8 induced serum gave a higher titer of 160 (Fig. 5B).

### References

Bosch, F.X., M. Orlinch, H.D. Klenk, and R. Rott. 1979. The structure of the hemagglutinin, a determinant for the pathogenicity of influenza viruses. Virology. 95:197-207.
He, Q., Q. Du, S. Lau, I. Manopo, L. Lu, S.W. Chan, B.J. Fenner, and J. kwang. 2005. Characterization of monoclonal antibody against SARS coronavirus nucleocapsid antigen and development of an antigen capture ELISA. J. Virol. Methods. 127:46-53.
Hsu, C., Y. Ho, K. Wang, and Y. Hu. 2004. Investigation of optimal transduction conditions for baculovirus-mediated gene delivery into mammalian cells. Biotechno/. Bioeng. 88:42-51.
Kawaoka, Y. and R.G. Webster. 1988. Sequence requirements for cleavage activation of influenza virus hemagglutinin expressed in mammalian cells. Proc Natl Acad Sci USA. 85:324-328.
Lu, L., Q. Du, and N. Chejanovsky. 2003. Reduced expression of the immediate-early protein 1E0 enables efficient replication of Autographa californica multiple nucleopolyhedrovirus in poorly permissive Spodoptera littoralis cells. J. Virol. 77:535-545.
Lu, L., H. Wang, I. Manopo, L. Yu, and J. Kwang. 2005. Baculovirus-mediated promoter assay and transcriptional analysis of white spot syndrome virus orf427 gene. Virol. J. 2:71.
Takada, A., N. Kuboki, K. Okazaki, A. Ninomiya, H. Tanaka, H. Ozaki, S. Itamura, H. Nishimura, M. Enami, M. Tashiro, K.F. Shortridge, and H. Kida. 1999. Avirulent Avian influenza virus as a vaccine strain against a potential human pandemic. J. Virol 73:8303-8307.
Wood, J.M., Y. Kawaoka, L.A. Newberry, E. Bordwell, and R.G. Webster. 1985. Standarization of inactivated H5N2 influenza vaccine and efficacy against lethal A/chicken/Pennsylvania/1370/83 infection. Avian Dis. 29:867-872.
Rowe T, Abernathy RA, Hu-Primmer J, Thompson WW, Lu X, Lim W, Fukuda K, Cox NJ, Katz JM. 1999 Detection of antibody to avian influenza A (H5N1) virus in human serum by using a combination of serologic assays. J Clin Microbiol. 1999 Apr;37(4):937-43.

## Claims

1. A vaccine for use in method for the treatment or prevention of a disease in a subject, wherein said disease is associated with an avian influenza virus, and wherein said vaccine comprises an expression vector comprising a nucleic acid encoding a hemagglutinin peptide, such that in use said hemagglutinin peptide is expressed by said expression vector in said subject, wherein the expression vector further comprises an *ie1* promoter from white spot syndrome virus operably linked to the nucleic acid encoding said hemagglutinin peptide.

2. The vaccine for use according to claim 1, wherein the expression vector comprises a baculovirus expression vector.

3. The vaccine for use according to according to any one of claims 1 to 2, wherein the subject is selected from the group comprising human, avian or porcine.

4. The vaccine for use according to any one of claims 1 to 3, wherein the avian influenza virus is an H5N1 subtype of avian influenza virus.

5. The vaccine for use according to any one claims 1 to 4, wherein the hemagglutinin peptide comprises an amino acid sequence derived from an H5N1 subtype of avian influenza virus.

6. An expression vector, wherein said expression vector comprises:
(a) a nucleic acid encoding an antigenic peptide; and
(b) an *ie1* promoter from white spot syndrome virus operably linked to the nucleic acid encoding an antigenic peptide
wherein said antigenic peptide comprises a hemagglutinin peptide comprising an amino acid sequence derived from an H5N1 subtype of avian influenza virus.

7. A host cell comprising the expression vector of claim 6.

8. A composition comprising an immunopotentiating agent selected from the group consisting of the expression vector referred to in any one of claims 1 to 5, the expression vector according to claim 6 or the host cell according to claim 7, together with a pharmaceutically acceptable carrier, diluent or excipient, and optionally an adjuvant.

9. The expression vector referred to in any one of claims 1 to 5, the expression vector according to claim 6, the host cell according to claim 7, the composition according to claim 8.
for use in a method for the modulation of an immune response.

10. The expression vector according to claim 6, the host cell according to claim 7 or the composition according to claim 8 for use in a method for the treatment or prevention of a disease associated with an avian influenza virus.

## Patentansprüche

1. Impfstoff zur Verwendung in einem Verfahren zur Behandlung oder Vorbeugung einer Krankheit an einem Subjekt, wobei die besagte Krankheit mit einem Vogelgrippe-Virus verbunden ist, und wobei der besagte Impfstoff einen Expressionsvektor umfasst, der eine Nukleinsäure umfasst, die ein Hämagglutinin-Peptid codiert, sodass das besagte Hämagglutinin-Peptid im Einsatz am besagten Subjekt durch den besagten Expressionsvektor ausgedrückt wird, wobei der Expressionsvektor darüber hinaus einen *ie1* Promotor des Weißpünktchenkrankheit-Virus umfasst, der funktional mit der Nukleinsäure zum Codieren des besagten Hämagglutinin-Peptids verbunden ist.

2. Impfstoff zur Verwendung nach Anspruch 1, wobei der Expressionsvektor einen Baculovirus-Expressionsvektor umfasst.

3. Impfstoff zur Verwendung nach irgendeinem der Ansprüche 1 bis 2, wobei das Subjekt aus einer Gruppe ausgewählt wird, die Menschen, Vögel oder Schweine umfasst.

4. Impfstoff zur Verwendung nach irgendeinem der Ansprüche 1 bis 3, wobei der Vogelgrippe-Virus ein H5N1-Subtyp des Vogelgrippe-Virus ist.

5. Impfstoff zur Verwendung nach irgendeinem der Ansprüche 1 bis 4, wobei das Hämagglutinin-Peptid eine Aminosäuresequenz umfasst, die von einem H5N1-Subtypen eines Vogelgrippe-Virus abgeleitet ist.

6. Expressionsvektor, wobei der besagte Expressionsvektor folgendes umfasst:
(a) eine Nukleinsäure, die ein Antigenpeptid codiert; und
(b) einen *ie1* Promotor des Weißpünktchenkrankheit-Virus, der funktional mit der Nukleinsäure zum Codieren eines Antigenpeptids verbunden ist wobei das besagte Antigenpeptid ein Hämagglutinin-Peptid umfasst, das eine Aminosäuresequenz umfasst, die von einem H5N1-Subtypen eines Vogelgrippe-Virus abgeleitet ist.

7. Wirtszelle, den Expressionsvektor aus Anspruch 6 umfassend.

8. Zusammensetzung, ein das Immunsystem stärkendes Mittel umfassend, das aus der Gruppe ausgewählt wird, die aus dem Expressionsvektor nach irgendeinem der Ansprüche 1 bis 5 besteht, aus dem Expressionsvektor nach Anspruch 6, oder der Wirtszelle nach Anspruch 7, zusammen mit einem pharmazeutisch annehmbaren Träger, Verdünnungsmittel oder Hilfsstoff, und optional einem Zusatz.

9. Expressionsvektor nach irgendeinem der Ansprüche 1 bis 5, Expressionsvektor nach Anspruch 6, Wirtszelle nach Anspruch 7, Zusammensetzung nach Anspruch 8 zur Verwendung in einem Verfahren zum Modulieren einer Immunantwort.

10. Expressionsvektor nach Anspruch 6, Wirtszelle nach Anspruch 7 oder Zusammensetzung nach Anspruch 8 zur Verwendung in einem Verfahren zur Behandlung oder Vorbeugung einer Krankheit in Verbindung mit einem Vogelgrippe-Virus.

## Revendications

1. Vaccin pour une utilisation dans un procédé de traitement ou de prévention d'une maladie chez un sujet, où ladite maladie est associée à un virus de la grippe aviaire, et où ledit vaccin comprend un vecteur d'expression comprenant un acide nucléique codant pour un peptide de l'hémagglutinine, de sorte que lors de l'utilisation, le dipeptide de l'hémagglutinine est exprimé par ledit vecteur d'expression chez ledit sujet, où le vecteur d'expression comprend en outre un promoteur *ie1* provenant du virus du syndrome des taches blanches lié de façon fonctionnelle à l'acide nucléique codant pour ledit peptide de l'hémagglutinine.

2. Vaccin pour une utilisation selon la revendication 1, où le vecteur d'expression comprend un vecteur d'expression de baculovirus.

3. Vaccin pour une utilisation selon l'une quelconque des revendications 1 à 2, où le sujet est choisi dans le groupe comprenant un être humain, un oiseau ou un porc.

4. Vaccin pour une utilisation selon l'une quelconque des revendications 1 à 3, où le virus de la grippe aviaire est un sous-type H5N1 du virus de la grippe aviaire.

5. Vaccin pour une utilisation selon l'une quelconque des revendications 1 à 4, où le peptide de l'hémagglutinine comprend une séquence d'acides aminés dérivée d'un sous-type H5N1 du virus de la grippe aviaire.

6. Vecteur d'expression, où ledit vecteur d'expression comprend :
(a) un acide nucléique codant pour un peptide antigénique ; et
(b) un promoteur *ie1* provenant du virus du syndrome des taches blanches lié de façon fonctionnelle à l'acide nucléique codant pour un peptide antigénique où ledit peptide antigénique comprend un peptide de l'hémagglutinine comprenant une séquence d'acides aminés dérivée d'un sous-type H5N1 du virus de la grippe aviaire.

7. Cellule hôte comprenant le vecteur d'expression selon la revendication 6.

8. Composition comprenant un agent immunopotentialisateur choisi dans le groupe constitué du vecteur d'expression auquel il est fait référence dans l'une quelconque des revendications 1 à 5, du vecteur d'expression selon la revendication 6 ou de la cellule hôte selon la revendication 7, conjointement avec un support, un diluant ou un excipient pharmaceutiquement acceptable, et éventuellement un adjuvant.

9. Vecteur d'expression auquel il est fait référence dans l'une quelconque des revendications 1 à 5, vecteur d'expression selon la revendication 6, cellule hôte selon la revendication 7, composition selon la revendication 8 pour une utilisation dans un procédé de modulation d'une réponse immunitaire.

10. Vecteur d'expression selon la revendication 6, cellule hôte selon la revendication 7 ou composition selon la revendication 8 pour une utilisation dans un procédé de traitement ou de prévention d'une maladie associée à un virus de la grippe aviaire.


### SEQUENCE LISTING

<110> Temasek Lifesciences Laboratory
<120> Biomolecule surface display and uses thereof
<130> 747345M
<160> 10
<170> PatentIn version 3.2
<210> 1
   <211> 31
   <212> DNA
   <213> Artificial
<220>
   <223> synthetic
<400> 1
   cgcggatcca tgaagtgcct tttgtactta g 31
<210> 2
   <211> 30
   <212> DNA
   <213> artificial
<220>
   <223> synthetic
<400> 2
   cccaagcttc caacacacta ttgcaatgaa 30
<210> 3
   <211> 33
   <212> DNA
   <213> artificial
<220>
   <223> synthetic
<400> 3
   tcccccgggg gatggttggc tacgtatact ccg 33
<210> 4
   <211> 29
   <212> DNA
   <213> artificial
<220>
   <223> synthetic
<400> 4
   cgcggatccg gtttcggacc gagatccgc 29
<210> 5
   <211> 42
   <212> DNA
   <213> artificial
<220>
   <223> synthetic
<400> 5
   ataagaatgc ggccgctatg gagaaaacag tgcttcttct tg 42
<210> 6
   <211> 41
   <212> DNA
   <213> artificial
<220>
   <223> synthetic
<400> 6
   ccgctcgagc ggttaaatgc aaattctgca ttgtaacgat c 41
<210> 7
   <211> 34
   <212> DNA
   <213> artificial
<220>
   <223> synthetic
<400> 7
   tccctacgta tcaattttat gtggctaatg gaga 34
<210> 8
   <211> 37
   <212> DNA
   <213> artificial
<220>
   <223> synthetic
<400> 8
   acgcgtcgac cttgagtgga gagagagcta gttataa 37
<210> 9
   <211> 36
   <212> DNA
   <213> artificial
<220>
   <223> synthetic
<400> 9
   cgcggatccg atggagaaaa cagtgcttct tctttg 36
<210> 10
   <211> 30
   <212> DNA
   <213> artificial
<220>
   <223> synthetic
<400> 10
   cggggtaccc tctctttgag gggtatttct 30